# EUROPEAN PATENT APPLICATION

(11) **EP 3 485 872 A1**
(43) Date of publication of application: **22.05.2019**
(21) Application number: 17201796.4
(22) Date of filing: 15.11.2017
(51) Int. Cl.: A61K 8/73, A61Q 19/00, A61Q 19/08, A61K 8/19, A61K 31/728, A61K 33/00, A61P 17/06, A61P 17/18, A61P 17/00

(54) **COMPOSITIONS COMPRISING HYALURONIC ACID AND H2**

(71) Applicant: Peters, Jennifer, 40545 Düsseldorf (DE); Waldmann, Barbara, 40212 Düsseldorf (DE)
(72) Inventor: Peters, Jennifer, 40545 Düsseldorf (DE); Waldmann, Barbara, 40212 Düsseldorf (DE)
(74) Representative: Maiwald Patent- und Rechtsanwaltsgesellschaft mbH

(57) **Abstract**

The present invention relates to a composition comprising hyaluronic acid and H₂ and its cosmetic as well as its medical use. In particular, the composition is used for treating cosmetic and medical skin alterations. Non-therapeutic methods comprising the administration of a composition comprising hyaluronic acid and H₂ are also encompassed.

## Description

### FIELD OF THE INVENTION

The present invention relates to a composition comprising hyaluronic acid and H₂ and its cosmetic as well as its medical use. In particular, the composition is used for treating cosmetic and medical skin alterations. Non-therapeutic methods comprising the administration of a composition comprising hyaluronic acid and H₂ are also encompassed.

### BACKGROUND OF THE INVENTION

Skin aging results from a multifactorial process driven by intrinsic factors to the body such as genetics or the formation of radicals within cells and extrinsic factors such as mechanical stress, environmental and chemical toxins, pollutants, smoking or sun exposure. Even though intrinsic and extrinsic skin aging are distinct processes, they share similarities in molecular mechanisms. Reactive oxygen species (ROS) such as peroxide, hydroxide, superoxide, nitrogen oxide anion and peroxynitrite for example arise within the cell mainly as part of the oxidative cell metabolism but can also result from contact with chemical reagents or UV exposure. These free oxygen radicals are capable of DNA damage, destruction of protein cross-links within the cell membrane and exert an impact on peroxidases, thereby leading to premature cell death.

The strong cellular oxidizing potential of excess ROS also leads to oxidative stress since cells are not able to readily detoxify the reactive species and repair the resulting damage. Persistent oxidative stress is widely accepted as one of the causes of lifestyle-related diseases such as cancer and the aging process.

Aging of the skin can be characterized for example by coarseness, dryness, puffiness, fine lines, wrinkles, itching, slackness, visible vascular dilations, large pores and pigmentation (e.g. age spots).

Typical compounds used in skin care and skin treatment are hyaluronic acid, retinoids including vitamin A, other vitamins and/or amino acids. The afore-mentioned compounds are often used in combination, e.g. as part of the so called "mesotherapy". Hyaluronic acid is an anionic non-sulfated glycosaminoglycan, which is distributed throughout the human body in connective, epithelial, and neural tissues. While hyaluronic acid has been shown to bind water, ensure good hydration and boost the skin, the effect of amino acids and vitamins seems to be limited or even non-existing. In fact, vitamins need specific transporters and co-enzymes in order to be able to enter a cell. It can be assumed that in the course of common skin aging treatments, vitamins are mostly incapable of entering the cells and exert their antioxidative function. This assumption is supported by the fact that it is highly controversial whether vitamin preparations which are administered dermally exert any effect on skin appearance and or vitality [Jäger et al.]. Therefore, existing skin aging treatments are mostly transient and superficial. Furthermore, certain vitamin mixtures were shown to have harmful effects, causing for instance apoptosis in human skin fibroblasts [Jäger et al.] and vitamin C and amino acids may even give rise to certain allergies, which in turn result in redness.

Therefore, there is a need to provide a composition capable of effective, long-lasting treatment of the above outlined symptoms of aging skin without the disadvantages of existing treatments such as for example allergic reactions.

### OBJECTIVES AND SUMMARY OF THE INVENTION

In order to overcome this need the application provides a composition comprising hyaluronic acid and H₂. In particular the invention describes compositions comprising hyaluronic acid and H₂-enriched saline.

In contrast to antioxidants such as vitamins or contrary to amino acids, H₂ is able to rapidly diffuse into cells, even into cell organelles such as the mitochondria or the nucleus and therefore distributes very well throughout the body. H₂ reduces cytotoxic oxygen radicals e.g. hydroxide and peroxynitrite [Ohsawa et al.] but neither disturbs metabolic redox reactions nor affects ROS that function in cell signaling and should therefore have little adverse effects [Salganik; Sauer et al.; Liu et al.]. Furthermore, H₂ prevents oxidative stress and shows an anti-apoptotic and anti-inflammatory effect [Zhang et al.]. Unlike with other substances showing antioxidative effects such as vitamin E, the inventors found that the above described properties of H₂ could be used in combination with hyaluronic acid in order to preclude apoptosis and thus, the skin aging process and symptoms thereof without raising allergic reactions.

Hence a first aspect of the invention relates to a composition comprising hyaluronic acid and H₂.

In one embodiment the present invention relates to a composition comprising hyaluronic acid and H₂-enriched saline. In a further embodiment the present invention relates to a composition comprising hyaluronic acid and H₂-enriched saline in a ratio of about 5:1 to about 1:5. In yet another embodiment the present invention relates to a composition comprising hyaluronic acid, H₂ and autologous plasma.

H₂ which is dissolved in solution may be safer and is easier for administration.

A second aspect of the invention refers to the use of the composition as described in any of the above embodiments for treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual.

Accordingly, the invention also refers to a method of treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual, the method comprising the step of administering to an individual an effective amount of the composition as described above.

Since H₂ shows an anti-inflammatory effect and preserves cells, one embodiment of the invention relates to a method of treating redness, pigmentation and/or for reducing the size of skin pores in an individual, the method comprising the step of administering to an individual an effective amount of the composition as described above.

As outlined above, it is shown that H₂ shows an antioxidative effect [Ohsawa et al.] and was implicated to play a role in oxidative stress related diseases such as ischemia-reperfusion (I/R) injury, lipid and glucose metabolism in patients with type 2 diabetes impaired glucose tolerance, Alzheimer's and Parkinson's disease, kidney transplantation, acute pancreatitis and other oxidative stress related diseases [Shi et al.]. However, despite the clinical importance of oxidative damage to cells, antioxidants have been of limited therapeutic success.

However, the inventors found that a composition comprising hyaluronic acid and H₂ can be used as a medicament. Thus, in a third aspect the invention relates to said composition for use as a medicament.

Since H₂ shows an anti-inflammatory effect and preserves cells, one embodiment of the invention further relates to a composition comprising hyaluronic acid and H₂ for use in treating erythema, eczema, allergic eczema, utopias, and neurodermatitis, wherein the composition is administered to an individual.

### FIGURE LEGENDS

**Figure 1A****.** Before administration of the composition as described in Example 1; B. 3 weeks after the last administration as described in Example 1.
**Figure 2A****.** Before administration of the composition as described in Example 2; **B.** After administration of the composition as described in Example 2.
**Figure 3A****.** Before administration of the composition as described in Example 6; **B.** After administration of the composition as described in Example 6.
**Figure 4A****.** Before administration of the composition as described in Example 7; **B.** After administration of the composition as described in Example 7.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The term "about" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

A first aspect of the invention relates to a composition comprising hyaluronic acid and H₂.

The term "hyaluronic acid" is synonymous with "HA", "hyaluronic acid polymer", "hyaluronate", "hyaluronan" and "hyaluronan poylmer" and refers to a non-sulfated glycosaminoglycan comprising disaccharides of D-glucuronic acid and N-acetyl-D-glucosamine linked by a glucuronidic β-bond and pharmaceutically acceptable salts thereof. Examples of acceptable salts comprise sodium salts, potassium salts, magnesium salts, calcium salts, and combinations thereof. Hyaluronic acid can be purified from animal or non-animal sources [Boeriu et al.; Sze JH et al.] or can be obtained by chemical synthesis [e.g. Lu et al.]. Hyaluronic acid preparation can be obtained commercially for example from Galderma e.g. Restylane Skinbooster™, from Allergan e.g. Juvéderm® Volbella and Voluma line. The term "hyaluronic acid" as referred to herein also comprises modified hyaluronic acid.
Hyaluronic acid polymers ranging from the size of about 30,000 Da to about 60,000 Da are referred to as being of "low molecular weight", while hyaluronic acid polymers having a size of at least 1000 kDa are considered "high molecular weight".

As used herein the term "crosslinked" refers to the intermolecular bonds joining the individual polymers or monomer chains into a stable structure such as a gel or hydrogel. Hyaluronic acid polymers may be crosslinked using dialdehydes and disulfides crosslinking agents including but not limited to multifunctional PEG-based crosslinking agents, divinyl sulfones, diglycidyl ethers, bis-epoxides and biscarbodiimide.

The term "non-crosslinked" consequently refers to the absence of such intermolecular bonds joining the individual polymers or monomer chains. Non-crosslinked hyaluronic acid polymers are water soluble and generally remain fluid. As such, non-crosslinked hyaluronic acid polymers are often mixed with a lubricant to facilitate extrusion through a fine needle.

The term "H₂" as referred to herein is used synonymously with "hydrogen". H₂ being the molecular chemical formula for hydrogen diatomic gas meaning H₂ is not bound to any further chemical element.
There are several different ways to administer H₂ including but not limited to inhaling hydrogen gas, oral or topical application of H₂ dissolved in water, injection or topical administration of H₂ dissolved in saline. Preferably H₂ is dissolved in solution, more preferably in aqueous solution, even more preferably in saline.

Thus, in one embodiment the invention relates to a composition comprising hyaluronic acid and H₂-enriched saline.

"H₂-enriched saline" refers to a sodium chloride solution which is supplemented with H₂. Preferably, the saline solution contains 0.9% sodium chloride. A saline solution containing 0.9% sodium chloride corresponds in its salt content to the salt content of blood and can therefore be described also as physiological saline, which is non-irritating. H₂-enriched saline may be produced by dissolving H₂ for at least 2 hours, preferably 2 hours to 6 hours under high pressure such as 0.4 mPa. Gas chromatography may be used to measure or confirm H₂ content in saline [Oshawa et al.]. The H₂-enriched saline can be stored under atmospheric pressure at 4°C in an aluminum container with no dead volume. Alternatively, the H₂-enriched saline can also be stored under atmospheric pressure at 4°C in a glass ampule. Preferably, the saline, which H₂ is dissolved in, is sterilized and the container in which the saline is kept is autoclaved. H₂-enriched saline is preferably stored in sterile glass ampules. It can further be sterilized prior to use.

Typically, the H₂-enriched saline as referred to herein has a H₂-concentration of about 0.1 mmol/L to about 1.2 mmol/L. In a preferred embodiment the H₂-enriched saline has a H₂-concentration of about 0.4 mmol/L to about 1.0 mmol/L. In a more preferred embodiment the H₂-enriched saline has a H₂-concentration of about 0.6 mmol/L to about 0.8 mmol/L. In a most preferred embodiment the concentration of the H₂ in H₂-enriched saline is about 0.6 mmol/L. In an utmost preferred embodiment the concentration of H₂ in the H₂-enriched saline is 0.6 mmol/L. Gas chromatography may be used to measure or confirm H₂ content in saline [Oshawa et al.].

In one embodiment, the composition comprising hyaluronic acid and H₂-enriched saline comprises H₂-enriched saline in a concentration of about 0.01 ml/ml composition volume to about 0.99 ml/ml composition volume or of about 0.01 ml/ml to about 0.9 ml/ml composition volume. In a preferred embodiment H₂-enriched saline is present in a concentration of about 0.2 ml/ml composition volume to about 0.6 ml/ml composition volume. In a most preferred embodiment H₂-enriched saline is present in a concentration of about 0.33 ml/ml composition volume, preferably in a concentration of 0.33 ml/ml composition volume. However, it is noted that even at high concentration, H₂ shows no cytotoxicity.

In some embodiments, the hyaluronic acid is non-crosslinked. Non-crosslinked hyaluronic acid might be preferred when the composition is administered topically e.g. as part of a cream or a serum.

In some embodiments, the hyaluronic acid is crosslinked. Crosslinked hyaluronic acid might be preferred for injection.

In some embodiments, the disclosed compositions comprise hyaluronic acid of low and higher molecular weight. In a preferred embodiment at least 50%, at least 60%, at least 70%, at least 80% or at least 90% of the hyaluronic acid is of low molecular weight. In another preferred embodiment at least 90% of the hyaluronic acid is of low molecular weight. In yet another preferred embodiment at least 90% of the hyaluronic acid is of low molecular weight and up to 10% are of high molecular weight. In a more preferred embodiment 90% of hyaluronic acid is of low molecular weight and 10% of hyaluronic acid is of high molecular weight.

In some embodiments, the hyaluronic acid is provided at an initial concentration of at least about 12 mg/ml, preferably about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml or about 28 mg/ml.

In another embodiment the hyaluronic acid and H₂-enriched saline is present in a ratio of about 5:1 to about 1:5. In a preferred embodiment the hyaluronic acid and H₂-enriched saline is present in a ratio of about 3:1 to about 1:1. In a most preferred embodiment the hyaluronic acid and H₂-enriched saline are present in a ratio of about 2:1. In an utmost preferred embodiment the hyaluronic acid and H₂-enriched saline are present in a ratio of 2:1.

In some embodiments, the disclosed compositions may optionally also comprise further compounds such as antioxidants, anti-inflammatory agents, an anti-irritant agent, an anesthetic agent, a vascoconstrictor, a vasodilator, an anti-acne agent, a pigmentation agent, an anti-pigmentation agent, or a moisturizing agent.

In a further embodiment, the antioxidant is selected from the group of polyol, flavonid, phytoalexin, ascorbic acid, tocopherol, tocotrienol, a lipoic acid, a carotenoid and melatonin, or any combination thereof.
In a further embodiment the anti-inflammatory agent is selected from the group of dexamethasone, prednisolone, corticosterone, budesonide, estrogen, sulfasalazine, mesalamine, cetirizine, diphenylhydramine, antipyrine, methyl salicylate, loratadine, thymol, carvacrol, bisabolol, allantoin, eucalyptol, phenazone, propyphenazone, a nonsteroidal anti-inflammatory drug NSAID, or any combination thereof.
In yet a further embodiment the anti-itching agent is selected from the group of methyl sulphonyl methane, sodium bicarbonate, calamine, allantoin, kaolin, peppermint, tea tree oil, camphor, menthol, and hydrocortisone, or any combination thereof.
In another embodiment, the anti-cellulite agent is selected from the group of forskolin or xanthine, or any combination thereof.
In yet another embodiment, the anti-scarring agent is selected from the group of IFN-γ, fluorouracil, poly(lactic-co-glycolic acid), methylated polyethylene glycol, polylactic acid, polyethylene glycol, or any combination thereof.
In yet another embodiment, the anesthetic agent is selected from the group of lidocaine, articaine, bupivacaine, cinchocaine, etidocaine, levobupivacaine, mepivacaine, piperocaine, prilocaine, ropivacaine, trimecaine, or any combination thereof.
In yet another embodiment, the vasoconstrictor is selected from the group of naphazoline, epinephrine, methoxamine, methylnorepinephrine, norepinephrine, oxymetazoline, phenylephrine, pseudoephedrine, synephrine, cirazoline, and xylometazoline, or any combination thereof.
In a further embodiment, the anti-hemorrhagic agent is an anti-fibrinolytic agent selected from the group of ε-aminocaproic acid, tranexamic acid, and a serpin, or any combination thereof. In a preferred embodiment, the disclosed further compounds are present in an amount of 0.001% (w/w) to about 5% (w/w).

In yet another embodiment, the composition comprises hyaluronic acid, H₂-enriched saline and autologous plasma.

The term "autologous plasma" refers to an individual's own blood plasma which *inter alia* comprises stem cells and anti-inflammatory components such as Interleukin 10 and TGF-beta and therefore further enhances the anti-inflammatory effect of the composition according to the invention.

In some embodiments the hyaluronic acid, the H₂-enriched saline and the autologous plasma are present in a ratio of about 3.3:1.6:1, preferably of 3.3:1.6:1.

In some embodiments of the invention, the disclosed compositions further comprise a pharmaceutically acceptable excipient such as a stabilizing agent, a bulking agent, a cryo-protective agent, a lyo-protectant, an additive, a vehicle, a carrier, a diluent or an auxiliary, a buffer component, a preservative, a tonicity adjuster, a salt, an osmolality adjusting agent, an emulsifying agent, a wetting agent, a flavoring agent or any combinations thereof. Further examples of pharmaceutically acceptable excipients can be found e.g. in Pharmaceutical Dosage Forms and Drug Delivery Systems [Howard et al.; Alfonso et al.; Hardman et al. and Rowe et al.]. In some embodiments the disclosed composition comprises acetate buffers, borate buffers, citrate buffers, neutral buffered salines, phosphate buffers, and phosphate buffered salines. The pH of the buffers may be adjusted to a physiologically acceptable pH in the range of about pH 5.0 to about pH 8.5. Preferably from about 6.5 to about 7.5, more preferably from about 7.0 to about 7.4, most preferably from about 7.1 to about 7.3.

Skin aging is *inter alia* accompanied by a thinning of the epidermal layer (e.g. degradation of collagen and elastin) which acts as a barrier to harmful stimuli. As a consequence the sum of instrinsic and also increased extrinsic harmful stimuli e.g. leads to the visible appearance of fine lines and wrinkles.

Thus, second aspect of the invention therefore relates to the use of the disclosed compositions for treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual.

In a preferred embodiment, the disclosed compositions are used for treating redness, pigmentation and/or for reducing the size of skin pores in an individual.

Moreover, the invention relates to a method of treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual, comprising the step of administering to said individual an effective amount of the disclosed composition.

Moreover, the invention relates to a method of treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual, comprising the step of administering to said individual an effective amount of the disclosed composition, provided that the method is non-therapeutic.

In a preferred embodiment the invention relates to a method of treating redness, pigmentation and/or for reducing the size of skin pores in an individual, the method comprising the step of administering to said individual an effective amount of the disclosed composition.

In a preferred embodiment the invention relates to a method of treating redness, pigmentation and/or for reducing the size of skin pores in an individual, the method comprising the step of administering to said individual an effective amount of the disclosed composition, provided that the method is non-therapeutic.

An "individual" is typically a mammal, preferably a human being of any age, gender and race.

The term "effective amount" refers to an amount of the disclosed composition which markedly improves the disclosed cosmetic symptoms of skin aging.

In another preferred embodiment, the disclosed compositions are administered to an individual repeatedly. Preferably the disclosed compositions are administered once, twice or three times, more preferably at least three times. In some preferred embodiments the disclosed compositions are administered four times, five times, six times, seven times, eight times, nine times, ten times, eleven times or twelve times or even up to twenty-four times.

In yet another preferred embodiment the treatment interval is about two to eight weeks, preferably about four to six weeks, most preferably four to six weeks. This means administration takes place about every two to eight weeks, preferably about every four to six weeks, most preferably every four to six weeks.

The duration of the total treatment will typically be determined based on the cosmetic effect desired by the individual to be treated and the body part or region to be treated. Typically, the treatment duration is about 1 month, about 3 months, about 6 months, about 12 months up to about 24 months. The administration of the disclosed compositions can be daily (including multiple administrations in one day), weekly, monthly or bimonthly.

Generally, H₂ can be provided as part of and be released from compounds such as borohydride and salts thereof. However, borohydride may have unwanted, negative effects on the skin and eyes. Contact with sodium borohydride for example can severely irritate and burn the skin and eyes with possible eye damage and very high exposure may affect the nervous system [New Jersey department of health and senior services]. Thus, in accordance with the present invention H₂ or H₂-enriched solution or emulsion, preferably H₂-enriched saline comprises H₂ which is not bound to any further chemical element and is therefore not part of any compound such as (sodium)borohydride.

In one embodiment, the disclosed compositions are administered topically. Preferably, the disclosed compositions are administered as a solution, oil, lotion, gel, serum, ointment, cream, slurry, slum, salve, or paste. The disclosed compositions may be monophasic or multiphasic.

The disclosed compositions may be administered by lightly massaging them into an area of the skin, preferably a targeted area in order to minimize delivery to skin surface not requiring treatment. Suitable applicators for topical administration may include but are not limited to applicator wands, cotton swabs, or any other suitable device.

Preferably all components of a composition including hyaluronic acid but excluding H₂-enriched solution or emulsion, preferably H₂-enriched saline are pre-mixed. H₂-enriched solution or emulsion, preferably H₂-enriched saline is then added to the pre-mix before administration to an individual, preferably immediately before administration to an individual. In a preferred embodiment the pre-mix and H₂-enriched solution or emulsion, preferably H₂-enriched saline are present in a ratio of about 5:1 to about 1:5 within the final composition. In a more preferred embodiment the pre-mix and H₂-enriched solution or emulsion, preferably H₂-enriched saline are present in a ratio of about 2:1 within the final composition. In a more preferred embodiment the pre-mix and H₂-enriched solution or emulsion, preferably H₂-enriched saline are present in a ratio of about 2:1 within the final composition. In one embodiment 1 ml of the pre-mix is combined with 0.5 ml of H₂-enriched saline.
In another preferred embodiment autologous plasma is added so that the ratio of pre-mix, H₂-enriched solution or emulsion, preferably H₂-enriched saline and autologous plasma is 3.3:1.6:1. In a more preferred embodiment 1 ml of the pre-mix is combined with 0.5 ml of H₂-enriched saline and 0.3 ml autologous plasma.

An example of a pre-mix is a serum comprising water, butylene glycol, phenoxyethanol, hyaluronic acid, preferably sodium hyaluronate, ethylhexylglycerin, potassium sorbate and Leuconostoc/radish root ferment filtrate. In a preferred embodiment, the exemplary serum is mixed with H₂ or H₂-enriched saline and administered to an individual. In a preferred embodiment hyaluronic acid, preferably sodium hyaluronate and H₂, preferably H₂-enriched saline are present in a ratio of 2:1 within the serum to be administered. In another preferred embodiment 1 ml hyaluronic acid, preferably sodium hyaluronate is mixed with 0.5 ml H₂-enriched saline within the serum to be administered.
An exemplary mixture of a cream may comprise water, C₁₂₋₁₅ alykl benzoate, glycerin, butyrospermum park II (shea) butter, decyl oleate, zea mays (corn) germ oil, caprylic/capric triglyceride, cetearyl alcohol, hydrogenated vegetable glycerides, potassium cetyl phosphate, cocoglycerides, tocopheryl acetate, phenoxethanol, hydrogenated palm glycerides, carbomer xanthan gum, ethylhexylglycerin, Leuconostroc/radish root ferment filtrate, alcohol, sodium hydroxide, pantolactone, ascorbyl palmitate, citric acid, ascorbic acid, hyaluronic acid, preferably sodium hyaluronate and H₂ or H₂-enriched saline. In a preferred embodiment, the cream comprises water, oxtyldodecanol, C₁₂₋₁₅ alykl benzoate, glycerin, butyrospermum park II (shea) butter, decyl oleate, zea mays (corn) germ oil, cetearyl alcohol, glyceryl stearate, Lactobacillus/Portulaca oleracea ferment extract, sorbitol, propanediol, potassium cetyl phosphate, cocoglycerides, dimethicone, tocopheryl acetate, phenoxethanol, hydrogenated palm glycerides, panthenol, squalane, carbomer, xanthan gum, ethylhexylglycerin, potassium sorbate, Leuconostoc/radish root ferment filtrate, alcohol, caprylic/capric triglyceride, baicalin, pantolactone, ascorbyl palmitate, citric acid, ascorbic acid, sodium hyaluronate and H₂ or H₂-enriched saline. Again all components may be combined as a pre-mix before adding H₂ or H₂-enriched saline.

In a further embodiment the disclosed compositions are administered by injection. A preferred embodiment is dermal injection. An even more preferred embodiment is dermal injection via a fine needle, preferably a fine needle with a gauge of from about 28 G to about 31 G, most preferably with a gauge of 29 G or 30 G. Another preferred embodiment is subcutaneous injection. Another even more preferred embodiment is subcutaneous injection with a stub needle, preferably a stub needle with a gauge of from 24 G to 26 G, most preferably with a gauge of 25 G.

The term "dermal injection" as used herein refers to the injection into the dermis, just below the epidermis.

The term "subcutaneous injection" as used herein refers to the injection into the subcutis directly below the dermis and epidermis.

In a preferred embodiment a composition comprising hyaluronic acid and H₂ or H₂-enriched saline in a 2:1 ratio is administered dermally or subcutaneously. In another preferred embodiment a composition comprising 1 ml hyaluronic acid and 0.5 ml H₂-enriched saline is administered dermally or subcutaneously.

In yet another preferred embodiment a composition comprising hyaluronic acid (initial concentration 12 mg/ml) and H₂ or H₂-enriched saline is administered dermally or subcutaneously. In a preferred embodiment hyaluronic acid and H₂ or H₂-enriched saline are present in a 2:1 ratio in said composition. In another preferred embodiment a composition comprising 1 ml hyaluronic acid (initial concentration 12 mg/ml) and 0.5 ml H₂-enriched saline is administered dermally or subcutaneously.

In another embodiment, the disclosed compositions are administered in an injection volume of about 0.5 ml to about 2 ml, preferably of about 1.5 ml.

A third aspect of the invention relates to the use of the disclosed compositions as a medicament.

In particular, the inventors found that the disclosed compositions can be used in the treatment of erythema, eczema, allergic eczema, utopias, and neurodermatitis.

Erythemas may include but are not limited to erythema ab igne, erythema chronicum migrans, erythema induratum, erythema infectiosum (fifth disease), erythema marginatum, erythema migrans, erythema multiforme, erythema nodosum, erythema toxicum, erythema elevatum diutinum, erythema gyratum repens, keratolytic winter erythema, and palmar erythem.

Eczemas may include but are not limited to atopic dermatitis, dyhidrotic eczema, hand eczema, nummular eczema, seborrheic dermatitis, stasis dermatitis, discoid eczema, pompholyx eczema, asteatotic eczema, and varcose eczema. Utopias may include atopy for example atopic eczema.

Allergic eczemas may include but are not limited to allergic dermatitis, contact dermatitis, and allergic contact dermatitis.

In a preferred embodiment, the disclosed compositions can be used in the treatment of neurodermatitis.

In a preferred embodiment, the disclosed compositions are administered repeatedly. Preferably the disclosed compositions are administered once, twice or three times, more preferably at least three times. In some preferred embodiments the disclosed compositions are administered four times, five times, six times, seven times, eight times, nine times, ten times, eleven times or twelve times or even up to twenty-four times.

In yet another preferred embodiment, initial administration takes place at least three times, followed by administration around every six months. In an even more preferred embodiment the disclosed compositions are initially administered three to five times, followed by administration every six months.

The duration of treatment will typically be determined based on the therapeutic effect desired by the individual and/or the practitioner. Typically, treatments are scheduled for about 1 month, about 3 months, about 6 months, about 12 months up to about 24 months. The administration of the disclosed compositions can be daily (including multiple administration in one day), weekly, monthly or bimonthly.

In one embodiment, the disclosed compositions are administered topically. Preferably, the disclosed compositions are administered as a solution, oil, lotion, gel, serum, ointment, cream, slurry, salve, or paste. The disclosed compositions may be monophasic or multiphasic.

Preferably all components of a composition including hyaluronic acid but excluding H₂ or H₂-enriched saline are pre-mixed. H₂ or H₂-enriched saline is then dissolved or added to the pre-mix before administration to an individual, preferably immediately before administration to an individual. In a preferred embodiment the pre-mix and H₂, preferably H₂-enriched saline are present in a ratio of about 5:1 to about 1:5 within the final composition. In a more preferred embodiment the pre-mix and H₂ or H₂-enriched saline are present in a ratio of about 1:1 within the final composition. In an even more preferred embodiment the pre-mix and H₂ or H₂-enriched saline are present in a ratio of 1:1 within the final composition. In an utmost preferred embodiment 1 ml of the pre-mix is combined with 1 ml of H₂-enriched saline.
In another preferred embodiment autologous plasma is added so that the ratio of pre-mix, H₂ or H₂-enriched saline and autologous plasma is 3:3:1.6:1. In a more preferred embodiment 1 ml of the pre-mix is combined with 1 ml of H₂-enriched saline and 0.6 ml autologous plasma.

An example of a pre-mix is a serum comprising water, butylene glycol, Lactobacillus/Portulaca oleracea ferment extract, phenoxyethanol, hyaluronic acid, preferably sodium hyaluronate, ethylhexylglycerin, potassium sorbate, leuconostoc/radish root ferment filtrate. In a preferred embodiment, the exemplary serum is mixed with H₂ or H₂-enriched saline and administered to an individual. In a preferred embodiment hyaluronic acid, preferably sodium hyaluronate and H₂ or H₂-enriched saline are present in a ratio of about 1:1. In a more preferred embodiment hyaluronic acid, preferably sodium hyaluronate and H₂ or H₂-enriched saline are present in a ratio of 1:1. In another preferred embodiment 1 ml hyaluronic acid, preferably sodium hyaluronate is mixed with 1 ml H₂-enriched saline.

In a further embodiment the disclosed compositions are administered by injection. A preferred embodiment is dermal injection via a fine needle, preferably a fine needle with a gauge of from about 28 G to about 31 G, most preferably with a gauge of 29 G or 30 G. Another preferred embodiment is subcutaneous injection with a stub needle, preferably a stub needle with a gauge of from 24 G to 26 G, most preferably with a gauge of 25 G.

In another embodiment, the disclosed compositions are administered in an injection volume of about 0.5 ml to about 2 ml, preferably of about 1.5 ml.

The disclosed invention and its embodiments can also be performed without hyaluronic acid.

Accordingly, in one embodiment, the present invention relates to a composition comprising H₂-enriched saline.

In another embodiment, the present invention relates to the use of H₂-enriched saline for treating fine lines, wrinkles, fibroblast depletions, vascular dilation, redness, pigmentation or scars of an individual and/or for reducing the size of the skin pores in an individual and/or for increasing smoothness, hydration, and elasticity of the skin of an individual, wherein the composition is administered to said individual.

In a further embodiment, the present invention relates to a method of treating fine lines, wrinkles, fibroblast depletions, vascular dilation, redness, pigmentation or scars of an individual and/or for reducing the size of the skin pores in an individual and/or for increasing smoothness, hydration, and elasticity of the skin of an individual, wherein the method comprises the step of administering to said individual an effective amount of H₂-enriched saline.

In another embodiment, the present invention relates to H₂-enriched saline for use as a medicament.

In yet another embodiment, the present invention relates to H₂-enriched saline for use in treating erythema, eczema, allergic eczema, utopias, and neurodermatitis, wherein the composition is administered to an individual.

### EXAMPLES

The following examples illustrate representative embodiments, but should not be construed as limiting to the present invention.

### Example 1:

### An injectable hyaluronic acid/H₂-enriched saline composition for treating wrinkles and increasing elasticity

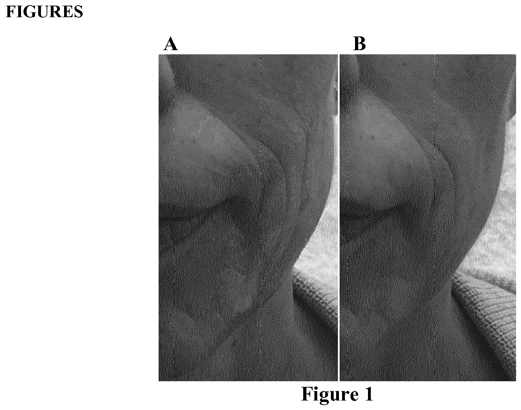

A composition comprising 1 ml hyaluronic acid (Skinbooster Vital Light, 12 mg/ml) and 0.5 ml H₂-enriched saline (0.6 mmol/L) was administered subcutaneously with a 25 G, 38 mm needle (Hypodermic CSH, TSK Laboratory) to each side of the face of a woman presented with wrinkles on the sides of her mouth. After three administrations at a three-week interval wrinkle depth was clearly reduced. This effect was long lasting since three weeks after the last treatment, wrinkle depth was still significantly reduced and the skin showed higher elasticity (see Figure 1A and 1B).

### Example 2:

### An injectable hyaluronic acid/H₂-enriched saline composition for treating pigmentation and fine lines

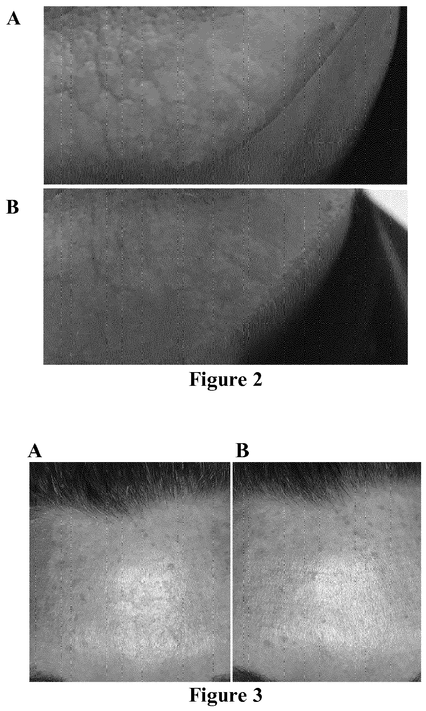

A composition comprising 1 ml hyaluronic acid (Skinbooster Vital Light, 12 mg/ml) and 0.5 ml H₂-enriched saline (0.6 mmol/L) was administered dermally with a 29 G or 30 G needle (Terumo Europe N.V. or TSK Laboratories). After three administrations at a three-week interval existing fine lines were markedly reduced (see Figure 2A and 2B). Pigmentation was reduced after dermally injecting the above composition five times.

### Example 3:

### An injectable or topically administered hyaluronic acid/H₂-enriched saline composition for reducing skin pore size and increasing hydration of the skin

A composition comprising 1 ml hyaluronic acid (Skinbooster Vital Light, 12 mg/ml) and 0.5 ml H₂-enriched saline (0.6 mmol/L) was administered dermally with a 29 G or 30 G needle (Terumo Europe N.V. or TSK Laboratories) to subjects. After three administrations at a three-week interval, skin pore size was visibly reduced and hydration of the skin was improved and lasted at least up to two weeks after the final treatment.

Furthermore, if a composition of hyaluronic serum (see Table 1) and H₂-enriched saline in a 1:1 ratio is topically administered two times a day over a period of 2 weeks the skin looked more hydrated with visibly less fine lines due to dryness. After a period of 5 weeks the skin showed a smoother complexion due to visibly finer skin pores.

### Table 1. Hyaluronic serum

Water
Butylene glycol
Lactobcillus/Portulaca oleracea ferment extract
Sodium hyaluronate
Ethylhexylglycerin
Potassium sorbate
Leuconostoc/Rasdish root ferment filtrate

### Example 4:

### An injectable or topically administered hyaluronic acid/H₂-enriched saline composition for treating redness and vascular dilation

Subjects showing redness of skin and minimal vascular dilations showed a marked decrease of these symptoms after a composition comprising 1 ml hyaluronic acid (Skinbooster Vital Light, 12 mg/ml) and 0.5 ml H₂-enriched saline (0.6 mmol/L) was administered subcutaneously with a 25 G, 38 mm needle (Hypodermic CSH, TSK Laboratory) into the perioral region (around the mouth). After three administrations at three-week intervals a reduction in redness and vascular dilation was observed.

Furthermore, if a composition of hyaluronic serum (see Table 1) and H₂-enriched saline in a 1:1 ratio is topically administered two times a day over a period of 6 weeks, a marked decrease of redness could be observed.

### Example 5:

### A hyaluronic acid/H₂-enriched saline composition for topical treatment of redness

First-degree sun burn leading to redness, swelling, pain and itching was topically treated with hyaluronic serum (see Table 1) and H₂-enriched saline in a 1:1 ratio three times in one day. The treatment led to a marked reduction of pain and resolution of swelling and a visible reduction of redness. The itching vanished directly after the administration of the hyaluronic acid/H₂-enriched saline composition while the swelling and pain improved after three hours.

### Example 6:

### H₂-enriched saline for topical treatment of redness

Sun burn leading to redness and itching was topically treated with H₂-enriched saline four times a day. The treatment led to a visible reduction of redness after one day (see Figures 3A and 3B). The itching vanished already 60 minutes after the first administration of H₂-enriched saline.

### Example 7:

### A hyaluronic acid/H₂-enriched saline composition for topical treatment of eczema

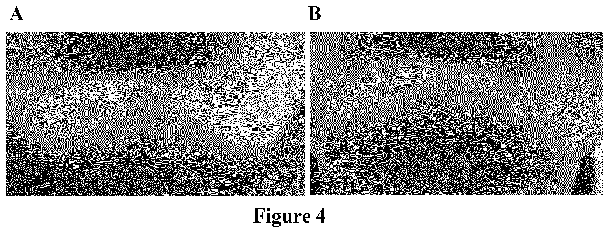

A composition comprising hyaluronic serum (see Table 1) and H₂-enriched saline in a 1:1 ratio was administered topically five times in one day to a woman presented with predominant primary skin lesions (pustules). The above treatment led to a marked healing and decline of pain, swelling, itching and redness (see Figure 4A and 4B).

In this context the above described composition is used as a medicament.

It is further noted that when injecting the compositions according to the Examples non-crosslinked hyaluronic acid (Skinbooster Vital Light, Galderma) was used at low concentrations of 12 mg/ml in order to ensure that the effects of the hyaluronic acid on the assessed symptoms were small or not detectable at all and therefore any observed effect can mainly be assigned to H₂-enriched saline.

The application further comprises the following embodiments:
Embodiment 1. A composition comprising hyaluronic acid and H₂.
Embodiment 2. The composition according to embodiment 1, wherein the composition comprises hyaluronic acid and H₂-enriched saline.
Embodiment 3. The composition according to embodiment 2, wherein the H₂-enriched saline has a H₂-concentration of about 0.1 mmol/L to about 1.2 mmol/L, preferably of about 0.6 mmol/L to about 0.8 mmol/L.
Embodiment 4. The composition according to any one of embodiments 2 or 3, wherein H₂-enriched saline is present in a concentration of at least 0.01 ml/ml composition volume.
Embodiment 5. The composition according to any one of embodiments 1 to 4, wherein the hyaluronic acid is non-crosslinked.
Embodiment 6. The composition according to any one of embodiments 1 to 4, wherein the hyaluronic acid is crosslinked.
Embodiment 7. The composition according to any one of embodiments 1 to 6, wherein at least 50%, at least 60%, at least 70%, at least 80%, preferably at least 90% of the hyaluronic acid is of low molecular weight.
Embodiment 8. The composition according to any one of embodiments 1 to 7, wherein the hyaluronic acid is provided at an initial concentration of about at least 12 mg/ml.
Embodiment 9. The composition according to any one of embodiments 1 to 8, wherein the hyaluronic acid is present in a concentration of about 3 mg/ml composition volume to about 30 mg/ml composition volume, about 5 mg/ml composition volume to about 20 mg/ml composition volume, about 6 mg/ml composition volume to about 15 mg/ml composition volume, preferably about 8 mg/ml composition volume.
Embodiment 10. The composition according to any one of embodiments 2 to 9, wherein hyaluronic acid and H₂-enriched saline are present in a ratio of about 5:1 to about 1:5.
Embodiment 11. The composition according to any one of embodiments 1 to 10 further comprising autologous plasma.
Embodiment 12. The composition according to embodiment 11, wherein hyaluronic acid, H₂-enriched saline and autologous plasma are present in a ratio of 3.3:1.6:1.
Embodiment 13. Use of the composition of any one of embodiments 1 to 12 for treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual, wherein the composition is administered to said individual.
Embodiment 14. The use of the composition according to embodiment 13 for treating redness, pigmentation and/or for reducing the size of skin pores in an individual, wherein the composition is administered to said individual.
Embodiment 15. The use of the composition according to any one of embodiment 13 or 14, wherein the composition is administered repeatedly.
Embodiment 16. The use of the composition according to embodiment 15, wherein the composition is administered at least three times.
Embodiment 17. The use of the composition according to embodiment 16, wherein four to six weeks lie in between the individual administrations.
Embodiment 18. The use of the composition according to any one of embodiments 13 to 17, wherein the composition is administered topically.
Embodiment 19. The use of the composition according to any one of embodiments 13 to 17, wherein the composition is administered by injection.
Embodiment 20. The use of the composition according to embodiment 19, wherein the composition is administered dermally.
Embodiment 21. The use of the composition according to embodiment 20, wherein the injections are delivered through a needle having a gauge of from about 28 G to about 31 G.
Embodiment 22. The use of the composition according to embodiment 19, wherein the composition is administered subcutaneously.
Embodiment 23. The use of the composition according to embodiment 22, wherein the injections are delivered through a needle having a gauge of from about 24 G to about 26 G.
Embodiment 24. The use of the composition according to any one of embodiments 19 to 23, wherein the injection volume is from about 0.5 ml to about 2 ml per injection.
Embodiment 25. A method of treating fine lines, wrinkles, fibroblast depletions, vascular dilation, redness, pigmentation or scars of an individual and/or for reducing the size of skin pores in an individual and/or for increasing smoothness, hydration, and elasticity in the skin of an individual, the method comprising the step of administering to said individual an effective amount of the composition of any one of embodiments 1 to 12.
Embodiment 26. A method of treating redness, pigmentation and/or reducing the size of skin pores in an individual, the method comprising the step of administering to said individual an effective amount of the composition of any one of embodiments 1 to 12.
Embodiment 27. The method of any one of embodiments 25 or 26, wherein the composition is administered repeatedly.
Embodiment 28. The method of embodiment 27, wherein the composition is administered at least three times.
Embodiment 29. The method of embodiment 28, wherein four to six weeks are in between the individual administrations.
Embodiment 30. The method of any one of embodiments 25 to 29, wherein the composition is administered topically.
Embodiment 31. The method of any one of embodiments 25 to 30, wherein the composition is administered by injection.
Embodiment 32. The method of embodiment 31, wherein the composition is administered dermally.
Embodiment 33. The method of embodiment 32, wherein the injections are delivered through a needle having a gauge of from about 28 G to about 31 G.
Embodiment 34. The method of embodiment 31, wherein the composition is administered subcutaneously.
Embodiment 35. The method of embodiment 34, wherein the injections are delivered through a needle having a gauge of from about 24 G to about 26 G.
Embodiment 36. The composition of any one of embodiments 1 to 12 for use as a medicament.
Embodiment 37. The composition of any one of embodiments 1 to 12 for use in treating erythema, eczema, allergic eczema, utopias, and neurodermatitis, wherein the composition is administered to an individual.
Embodiment 38. The composition for use according to embodiment 37, wherein the composition is administered repeatedly.
Embodiment 39. The composition for use according to embodiment 38, wherein the composition is administered at least three times.
Embodiment 40. The composition for use according to embodiment 39, wherein the composition is administered initially at least three times, followed by administration around every six months.
Embodiment 41. The composition for use according to any one of embodiments 37 to 40, wherein the composition is administered topically.
Embodiment 42. The composition for use according to any one of embodiments 37 to 40, wherein the composition is administered by injection.
Embodiment 43. The composition for use according to embodiment 42, wherein the composition is administered dermally.
Embodiment 44. The composition for use according to embodiment 43, wherein the injections are delivered through a needle having a gauge of from about 28 G to about 31 G.
Embodiment 45. The composition for use according to embodiment 42, wherein the composition is administered subcutaneously.
Embodiment 46. The composition for use according to embodiment 45, wherein the injections are delivered through a needle having a gauge of from about 24 G to about 26 G.
Embodiment 47. The composition for use according to any one of embodiment 42 to 46, wherein the injection volume is from about 0.5 ml to about 2 ml per injection.

### REFERENCES

Jäger C, Brenner C, Habicht J and Wallich R: Bioactive reagents used in mesotherapy for skin rejuvenation in vivo induce diverse physiological processes in human skin fibroblasts in vitro - a pilot study. Exp Dermatol. 2012, 21(1):72-5
Oshawa I, Ishikawa M, Takahashi K et al.: Hydrogen acts as a therapeutic antioxidant by selectively reducing cytotoxic oxygen radicals. Nat Med, 2007; 13: 688-94
Salganik RI: The benefits and hazards of antioxidants: controlling apoptosis and other protective mechanisms in cancer patients and the human population. J. Am. Coll. Nutr 2011; 20: 464-472
Sauer H, Wartenberg M, Hescheler J: Reactive oxygen species as intracellular messengers during cell growth and differentiation. Cell Physiol. Biochem 2001; 11: 173-86
Liu H, Colavitti R, Rovira, II and Finkel T: Redox-dependent transcriptional regulation. Circ. Res 2005, 97:967-74
Zhang Y, Sun Q, He B, Xiao J, Wang Z and Sun X: Anti-inflammatory effect of hydrogen-rich saline in a rat model of regional myocardial ischemia and reperfusion. Internat. Journal of Cardiology, vol. 148, no.1, pp. 91-95m 2011.
Shi Y, Liao KS, Zhao KL et al.: Hydrogen-rich saline attenuates acute renal injury in sodium taurocholate-induced severe acute pancreatitis by inhibiting ROS and NF-κB pathway. Mediators of Inflammation, vol. 2015
Boeriu CG, Springer J, Kooy FK et al.: Production Methods for Hyaluronan, Internat. Journal of Carbohydrate Chemistry, vol. 2013
Sze JH, Brownlie JC, Love CA: Biotechnological production of hyaluronic acid: a mini review, Bitoech (2016) 6:67
Lu X, Kamat MN, Huan L, and Huang X: Chemical synthesis of a hyaluronic acid decasaccharid, J Org Chem. 2009 October 16, 74(20): 7608-7617
Howard C. Ansel et al.: Pharmaceutical Dosage Forms and Drug Delivery Systems eds., Lippincott Williams & Wilkins Publishers, 7th ed. 1999
Alfonso R. Gennaro ed., Lippincott: Remington: The Science and Practice of Pharmacy, Lippincott, Williams & Wilkins, 10th ed. 2000
Joel G. Hardman et al.: Goodman & Gilman's The Pharmacological Basis of Therapeutics eds., McGraw-Hill Professional, 10th ed. 2001
Raymond C. Rowe et al.: Handbook of Pharmaceutical Excipients, APhA Publications, 4th edition 2003
New Jersey Department of Health and Senior Services: Hazardous substance fact sheet

## Claims

1. A composition comprising hyaluronic acid and H₂.

2. The composition according to claim 1, wherein the composition comprises hyaluronic acid and H₂-enriched saline.

3. The composition according to claim 2, wherein the H₂-enriched saline has a H₂-concentration of about 0.1 mmol/L to about 1.2 mmol/L, preferably of about 0.6 mmol/L to about 0.8 mmol/L.

4. The composition according to any one of claims 2 or 3, wherein H₂-enriched saline is present in a concentration of about at least 0.01 ml/ml composition volume, preferably in a concentration of about 0.33 ml/ml composition volume.

5. The composition according to any one of claims 1 to 4, wherein at least 50%, at least 60%, at least 70%, at least 80%, preferably at least 90% of the hyaluronic acid is of low molecular weight.

6. The composition according to any one of claims 1 to 5, wherein the hyaluronic acid is provided at an initial concentration of at least 12 mg/ml.

7. The composition according to any one of claims 2 to 6, wherein hyaluronic acid and H₂-enriched saline are present in a ratio of about 5:1 to about 1:5.

8. Use of the composition of any one of claims 1 to 7 for treating fine lines, wrinkles, fibroblast depletions, vascular dilation, redness, pigmentation or scars of an individual and/or for reducing the size of skin pores in an individual and/or for increasing smoothness, hydration, and elasticity of the skin of an individual, preferably for treating redness, pigmentation and/or for reducing the size of skin pores in an invidual, wherein the composition is administered to said individual.

9. The use of the composition according to claim 8, wherein the composition is administered repeatedly, preferably at least three time.

10. The use of the composition according to claim 9, wherein four to six weeks lie in between the individual administrations.

11. The use of the composition according to any one of claims 8 to 10, wherein the composition is administered topically or by injection, preferably be dermal or subcutaneous injection.

12. The composition of any one of claims 1 to 7 for use as a medicament.

13. The composition of any one of claims 1 to 7 for use in treating erythema, eczema, allergic eczema, utopias, and neurodermatitis, wherein the composition is administered to an individual.

14. The composition for use according to claim 13, wherein the composition is administered repeatedly, preferably at least three times.

15. The composition for use according to claim 13 or 14, wherein the composition is administered topically or by injection, preferably by dermal or subcutaneous inejction.
